# EUROPÄISCHE PATENTSCHRIFT

(11) **EP 2 626 128 B1**
(45) Veröffentlichungstag und Bekanntmachung des Hinweises auf die Patenterteilung: **19.11.2014**
(21) Anmeldenummer: 13159182.8
(22) Anmeldetag: 21.12.2010
(51) Int. Cl.: A61B 5/083, G01N 21/3504, G01N 21/39

(54) **Vorrichtung zur Infrarot-Absorptionsspektroskopie mit Vorkammer zur Homogenisierung eines Probegases**
Apparatus for infrared absorption spectroscopy with pre-chamber for homogenising a test gas
Dispositif de spectroscopie d'absorption à infrarouges avec préchambre pour l'homogénéisation d'un échantillon gazeux

(30) Priorität: 24.12.2009 DE 102009055320
(43) Veröffentlichungstag der Anmeldung: 14.08.2013
(62) Teilanmeldung aus: 10801578.5
(73) Patentinhaber: Humedics GmbH, 10625 Berlin (DE)
(72) Erfinder: Rubin, Tom, 12163 Berlin (DE); Heyne, Karsten, 14979 Großbeeren (DE)
(74) Vertreter: Sokolowski, Fabian

(56) Entgegenhaltungen:
- EP-A1- 2 311 552
- EP-A1- 2 347 818
- JP-A- 02 307 518
- JP-A- 54 128 863
- JP-A- 2007 090 262
- US-A- 3 649 199
- US-A- 5 042 501
- US-A1- 2005 008 542

## Beschreibung

Die Erfindung betrifft eine Messvorrichtung zur Untersuchung eines Probegases mittels Infrarot-Absorptionsspektroskopie gemäß Anspruch 1.

Seit Jahrhunderten ist bekannt, dass der Atemgeruch ein Indikator für eine mögliche Erkrankung sein kann - das prominenteste Beispiel ist der süßlich-fruchtige, durch Aceton verursachte Geruch bei Diabetes mellitus Typ 1. Selbst der Atem gesunder Menschen enthält mehrere hundert flüchtige chemische Verbindungen, sogenannte "volatile organic compounds" (VOCs), in geringer Konzentration (ppm- bis ppt-Bereich). Einige davon spielen bei physiologischen bzw. pathophysiologischen Prozessen eine wichtige Rolle. Liegt eine Erkrankung vor, steigt die Konzentration von bestimmten Spurengasen im Atem an. Bei manchen Erkrankungen lassen sich auch Gase nachweisen, die im gesunden Organismus nicht vorkommen. Somit bietet die Atemgasanalyse ein großes Potenzial für die klinische Diagnostik und Therapieüberwachung. Allerdings ist die Spurengas-Konzentration häufig so gering, dass sie mit den verfügbaren gasanalytischen Verfahren nicht ausreichend genau gemessen werden kann.

Es existieren hochempfindliche Nachweisverfahren, wie z.B. die Massenspektroskopie oder die FTIR-Spektroskopie in Mulitpass-Probenzellen. Allerdings sind solche Nachweisgeräte nicht direkt am Patienten einsetzbar und somit für den klinischen Alltag bedeutungslos. Das liegt auch daran, dass die Auswertung mehrere Tage dauert und durch den Transport der Probe unkalkulierbare Fehlerquellen auftreten. Mobile Aufbauten im Bereich der Infrarotabsorptionsspektroskopie mit Diodenlasern (z.B. Bleisalzlasern) als Lichtquellen sind ebenfalls seit mehreren Jahrzehnten im Einsatz, erreichten aber bis jetzt nicht die benötigte Stabilität über einen längeren Zeitraum für den empfindlichen Nachweis von Gasen, so dass auch hier der Einsatz auf die medizinische Grundlagenforschung beschränkt blieb.

Eine alternative Methode ist das sogenannte NDIRS-Verfahren (NDIRS - Nicht-Dispersive IR-Spektroskopie). Es weist Dichteschwankungen im Probegas nach, die durch Absorption von Infrarotlicht ausgelöst werden. Dieses Nachweisverfahren ist empfindlich und kann alle zweieinhalb Minuten eine Messung durchführen. Allerdings werden die Messergebnisse durch andere Gase wie z.B. Sauerstoff verfälscht, so dass diese Methode nur eingeschränkt im klinischen Alltag eingesetzt werden kann.

Eine weitere Methode wird von dem Unternehmen Oridion Systems Ltd. unter der Bezeichnung BreathlD® eingesetzt. Hier wird als Lichtquelle eine CO₂-Drucklampe verwendet. Allerdings ist diese Methode in ihrer Empfindlichkeit und Schnelligkeit durch auftretende Linienbreitenschwankungen (in der Lampe und im Probengas), geringe Lichtintensitäten und spektrale Fluktuationen stark eingeschränkt und liefert dadurch keine hochempfindlichen Messergebnisse in kurzer Zeit. Die NDIRS Methode und die Methode von Oridion Systems Ltd. eignen sich z.B. gut zum Nachweis des Bakteriums *Helicobacter pylori* im Patientenmagen. Die Anwesenheit des Bakteriums wird über einen erhöhten ¹³CO₂-Gehalt in der Ausatemluft nach Verabreichung eines ¹³C-markierten Diagnostikum qualitativ nachgewiesen.

Qualitative Testverfahren werden bedeutungslos, wenn der Test im gleichen Preissegment wie die Behandlung liegt. Eine weitere Strategie, um den einfachen und schnellen Nachweis von flüchtige chemische Verbindungen zu gewährleisten, ist der Einsatz von oberflächensensitiven Mikrochips, die spezielle Spurengase aus der Atemluft selektieren und binden. Damit ist ein empfindlicher Nachweis der flüchtige chemische Verbindungen möglich und die qualitative Entscheidung, ob der Patient erkrankt ist oder nicht, kann getroffen werden.

Der reine Nachweis einer Erkrankung ist aufschlussreich, gibt allerdings noch keine Auskunft über die geeignete Therapie. Daher liegt die Zukunft der Atemgasanalyse in der quantitativen Bestimmung des Erkrankungsgrades, die dem Arzt direkte Entscheidungshilfen für die Therapie an die Hand gibt. Wenn solche Tests einfach und schnell durchführbar sind und die Ergebnisse sofort für den Arzt in verständlicher Form vorliegen, kann sich der Test im klinischen Alltag durchsetzen.

Die Anforderungen an quantitative Atemgastests sind hoch: Zur eindeutigen Identifizierung der Spurengase ist eine hohe Selektivität und Nachweisempfindlichkeit erforderlich, da die Konzentration meist im ppm- bis ppb-Bereich liegt. Die exakte quantitative Bestimmung der Spurengasmenge muss gewährleistet sein. Außerdem sollten die Messungen online und in Echtzeit erfolgen, um eine aufwendige und fehleranfällige Probensammlung (z. B. in Beuteln oder im Seitenstrom) zu vermeiden. Für eine praktikable und wirtschaftliche Nutzung sind leichte Bedienbarkeit, Kompaktheit, Robustheit, geringer Wartungsaufwand und/oder ein günstiges Kosten-Nutzen-Verhältnis zu fordern. Diese hohen und vielfältigen Anforderungen können derzeit von keinem gasanalytischen Verfahren vollständig erfüllt werden.

Die ausgeatmete Luft von Menschen weist einen Kohlendioxidvolumenanteil von 2% bis 4% auf und wird in 10 bis 20 Atemzügen pro Minute, bei Kleinkindern und Neugeborenen sogar in 25 bis 50 Atemzügen pro Minute ausgeatmet. Die Atemdruck des Menschen beträgt etwa 50mbar bis maximal 160mbar, bei einem Volumen von etwa 0,5 Litern. Von der Atemluft gelangen nur etwa 70% in die Lunge, so dass auch nur in etwa 70% des Gasvolumens ein deutlich erhöhter Kohlendioxidanteil vorliegt. In dem restlichen Gasvolumen - dem Totraumvolumen - kann die Kohlendioxidkonzentration bis auf die Konzentration der Umgebungsluft von etwa 0,04% sinken. Dies führt dazu, dass die Kohlendioxidkonzentration der Atemluft um zwei Größenordnungen von 0,04% bis 4% schwanken kann. Kohlendioxidkonzentration von über 5% sind toxisch und können z.B. zu Kopfschmerzen und Krämpfen führen.

Die produzierte Kohlendioxidmenge hängt von dem individuellen Stoffwechsel jedes einzelnen Menschen ab. Es werden verschiedene Näherungsverfahren verwendet um die Kohlendioxidproduktion eines Menschen abzuschätzen. Die Größen, die dort eingehen, sind z.B. Gewicht und Körperoberfläche. Die Körperoberfläche wird meistens wiederum mit dem Gewicht und der Körpergröße abgeschätzt, so dass in der Medizin oftmals mit nur mäßig genauen Größen gerechnet wird, was eine quantitative Ergebnisauswertung stark einschränkt oder sogar unmöglich macht.

Zur direkten quantitativen Bestimmung von Stoffwechselvorgängen oder Metabolisierungen ist es erforderlich, die Dynamik des Prozesses zeitaufgelöst zu verfolgen, am besten in Echtzeit. Weist die Metabolisierungsdynamik eine Kinetik auf, die durch eine Differentialgleichung 1. Ordnung modelliert werden kann (Dynamik 1. Ordnung), so können durch Lösung der Differentialgleichung oder durch Anpassen einer Exponentialfunktion: y(t)=A*exp(-t/tau) das Maximum der Kinetik A und die Zeitkonstante tau bestimmt werden. Aus den Größen A und tau können dann quantitativ metabolische Parameter bestimmt werden. Die Auslösung der Metabolisierungsdynamik wird bestenfalls durch eine zeitlich kurze Initiation erreicht, z.B. die iv-Verabreichung eines Diagnostikums oder die Freisetzung eines Diagnostikums durch Belichtung/Bestrahlung.

Dauert die Freisetzung oder der Start der Dynamik länger als der Anstieg tau oder als ein Atemzug, so muss die Dynamik der Freisetzung gesondert bestimmt werden und von der Metabolisierungsdynamik entfaltet werden. Ein Beispiel für einen schnellen Metabolisierungsstart ist die iv-Verabreichung des Diagnostikums 13C-Methacetin im Bolus. Es wird mit dem Blut (ca 60 Herzschläge pro Minute) im Körper verteilt und gelangt in etwa einer Sekunde zur Leber, wo es metabolisiert wird zu Paracetamol und 13CO2. Der Start der Dynamik ist viel schneller als der Atemrhythmus und führt so zu einer Dynamik 1. Ordnung, die direkt ausgewertet werden kann. Wird das 13C-Methacetin allerdings oral verabreicht, führt die Adsorption im Magen zu einer Faltung der Dynamik mit der Magenadsorptionsdynamik, die die Dynamik signifikant verfälscht.

Um die Metabolisierungsdynamik in Echtzeit zu verfolgen, sollte jeder Atemzug mit sehr hoher Empfindlichkeit gemessen werden. Das bedeutet, dass die Atemluft in der Messkammer schnell ausgetauscht werden muss und dass in weniger als zwei Sekunden eine komplette Auswertung des Atemzuges erfolgt sein muss.

Ein Analyseverfahren, welches eine quantitative Bestimmung der Leberfunktion ermöglicht, ist in der WO 2007/000145 A2 beschrieben. Das Verfahren basiert auf einer Substratanflutung des zu metabolisierenden Substrates in der Leber und der Bestimmung der maximalen Umsatzgeschwindigkeit des Substrates, die Aussagen über die Leberfunktionskapazität eines Patienten ermöglicht.

Aus der WO 2007/107 366 A1 ist eine gattungsgemäße Vorrichtung zur spektroskopischen Analyse eines Gases bekannt, bei der eine Messkammer kontinuierlich mit einem Probegas durchströmt werden kann.

JP 2007-090262, JP 2-307518 und JP 54-128863 offenbaren für die Mischung von Gasen geeignete Vorrichtungen, in denen Teile des Gases nach Durchlaufen unterschiedlich langer Abzweigungen wieder zusammengeführt werden.

US 5,042,501 und US 2005/0008542-A1 offenbaren einer Analysevorrichtung vorgeschaltete Kammern zur Mischung von Atemluft.

Der vorliegenden Erfindung liegt die Aufgabe zugrunde, die aus der WO 2007/107 366 A1 bekannte Messvorrichtung zu verbessern.

Diese Aufgabe wird erfindungsgemäß durch eine Messvorrichtung mit den Merkmalen des Anspruchs 1 und ein Verfahren mit den Merkmalen des Anspruchs 32 gelöst. Ausgestaltungen der Erfindung sind in den Unteransprüchen angegeben.

Erfindungsgemäß ist eine Vorkammer vorgesehen, durch die das Probegas in die Messkammer strömt. Die Vorkammer ist dabei bevorzugt dazu ausgebildet, das Probegas auf eine bestimmte Temperatur zu erwärmen oder abzukühlen und dadurch den Wasserdampfgehalt des Probegases zu reduzieren.

In einer weiteren Ausgestaltung ist die Vorkammer alternativ oder zusätzlich bevorzugt dazu ausgebildet, den Wasserdampfgehalt des Probegases auf wenigstens 60% relative Luftfeuchtigkeit zu reduzieren. Die Reduktion des Wasserdampfgehaltes erfolgt vorzugsweise durch semipermeable Membranen, die ausschließlich einen Austausch von Wasserdampf (nicht jedoch von anderen Substanzen) erlauben. Die Luft außerhalb der Membran muss eine relative Luftfeuchtigkeit von weniger als 50% relative Luftfeuchtigkeit aufweisen. Ist der Wasserdampfgehalt außerhalb der Vorkammer geringer als innerhalb, so wird der Wasserdampfgehalt des die Vorkammer durchströmenden Probegases reduziert. Die Gesamtfläche der Membran bestimmt, wie viel Gasaustausch stattfinden kann.

Als Beispiel sei die Anwendung der Messvorrichtung für die Atemanalyse genannt, in der ein einzelner Atemzug (insbesondere ein vollständiger Atemzug) untersucht wird. Die Luftfeuchtigkeit in einem Atemzug beträgt oftmals mehr als 90% relative Luftfeuchtigkeit, die durch die semipermeable Membran in der Vorkammer auf weniger als 50% relative Luftfeuchtigkeit reduziert wird. Die aktive Fläche der semipermeablen Membran kann dabei z. B. mehr als 150 cm², insbesondere mehr als 200 cm² und ganz besonders mehr als 250 cm² betragen.

Erfindungsgemäß ist die Vorkammer dazu ausgebildet, das Probegas zu homogenisieren. Die Homogenisierung des einzelnen Probegases erfolgt durch verschiedene (mindestens zwei) Abzweigungen unterschiedlicher Länge und Durchmesser, die von Teilen des Probegases durchlaufen werden. Nach dem Bereich der Abzweigungen werden die Teile des Probegases wieder zusammengeführt. Dabei ist es wichtig, dass der Gesamtquerschnitt aller Abzweigungen (also die Summe der Querschnitte der einzelnen Abzweigungen) einen größeren oder gleich großen Strömungsquerschnitt wie der Rest der Messvorrichtung aufweist, damit durch die Abzweigungen kein erhöhter oder nur geringfügig erhöhter Druckwiderstand für den Fluss des Probegases in der Messvorrichtung erzeugt wird. Die Längen der verschiedenen Abzweigungen, durch die das Probegas fließt, sind so gewählt, dass Probegasvolumina einer bestimmten Volumengröße optimal durchmischt werden. Die Durchmischung findet rein passiv statt und nutzt einzig die Druckdifferenz zum Auslass der Messvorrichtung aus, die das Probegas zum Strömen verleitet.

Als Beispiel sei die Anwendung der Messvorrichtung für die Atemanalyse genannt, in der ein einzelner (insbesondere vollständiger) Atemzug homogenisiert wird. Das Ausatmen erzeugt den Druckunterschied, der das Probegas zum Strömen verleitet. Das durchschnittliche Volumen eines Atemzuges beträgt etwa 500 ml. Schon bei Abzweigungen mit drei verschiedenen Durchmessergrößen mit Verhältnissen d3:d2:d1 = 3:2:1 zeigt der laminare Volumenstrom unterschiedliche Geschwindigkeiten v3 < v2 < v1. Hält man den Gesamtdurchschnitt für die einzelnen Durchmessergrößen d1, d2 und d3 konstant, indem mehrere Abzweigungen mit Durchmessergrößen d1 und d2 gewählt werden, so fließt durch alle Abzweigungen mit gleichem Durchmesser etwa das gleiche Volumen (bei Vernachlässigung der Reibung). Durch die unterschiedlichen Strömungsgeschwindigkeiten des Probegases kann nun durch Auswahl der Abzweigungslänge die gewünschte Volumenmenge (z.B. 500 ml) gut durchmischt werden. Die Anzahl der Abzweigungen beträgt mindestens zwei. Je mehr Abzweigungen verwendet werden, desto homogener kann das Probegas durchmischt werden. Eine gute Durchmischung erlaubt eine präzisere und schnellere Messung von Gasbestandteilen im Probegas. Sie ist z.B. für hochpräzise Messungen mit Durchflussmesstechnik wichtig.

Die Durchmesser der einzelnen Abzweigungen sind vorzugsweise derart ausgewählt, dass eine zweite Abzweigung einen um mindestens 50 %, insbesondere mindestens 60 %, insbesondere mindestens 70 %, insbesondere mindestens 80 %, insbesondere mindestens 90 % und ganz besonders mindestens 100 % größeren Durchmesser aufweist als eine erste Abzweigung.

Die Erfindung wird nachfolgend unter Bezugnahme auf die Figuren der Zeichnungen anhand mehrerer Ausführungsbeispiele näher erläutert. Es zeigen:
- Fig. 1: ein Ausführungsbeispiel einer Messvorrichtung zur Untersuchung eines Probegases mittels Infrarot-Absorptionsspektroskopie;
- Fig. 2: die Messung einer ¹³CO₂ Absorptionslinie bei 2297,19 cm⁻¹ mittels der Messvorrichtung der Figur 1, wobei die Absorption in Abhängigkeit von der Frequenz in Wellenzahlen innerhalb eines ausgemessenen Spektralbereichs dargestellt ist;
- Fig. 3: die gleichzeitige Messung der ¹²CO₂ und der ¹³CO₂ Absorptionslinien im Verlauf von zwei aufeinander folgenden Atemzügen, wobei die Absorption zum einen gegen die Zeit und zum anderen gegen die Frequenz in Wellenzahlen dargestellt ist;
- Fig. 4: das Verhältnis der ¹³CO₂ zur ¹²CO₂ Konzentration im Messbereich zwischen 0 DOB und 300 DOB, wobei die Abzisse ein eingestelltes Konzentrationsverhältnis von Testgasen und die Ordinate durch die Messvorrichtung der Figur 1 gemessene Werte darstellt;
- Fig. 5: die ¹³CO₂ Zunahme eines Probanden nach Einnahme von 13C-markiertem Methacetin in Abhängigkeit von der Zeit;
- Fig. 6: Linienbreiten der CO₂ Absorptionslinien in Abhängigkeit von der CO₂ Konzentration des Probegases, bei gleichbleibendem Druck; und
- Fig. 7: eine schematische Darstellung eines Messablaufs zur Leberleistungsbestimmung unter Verwendung der Messvorrichtung der Figur 1.

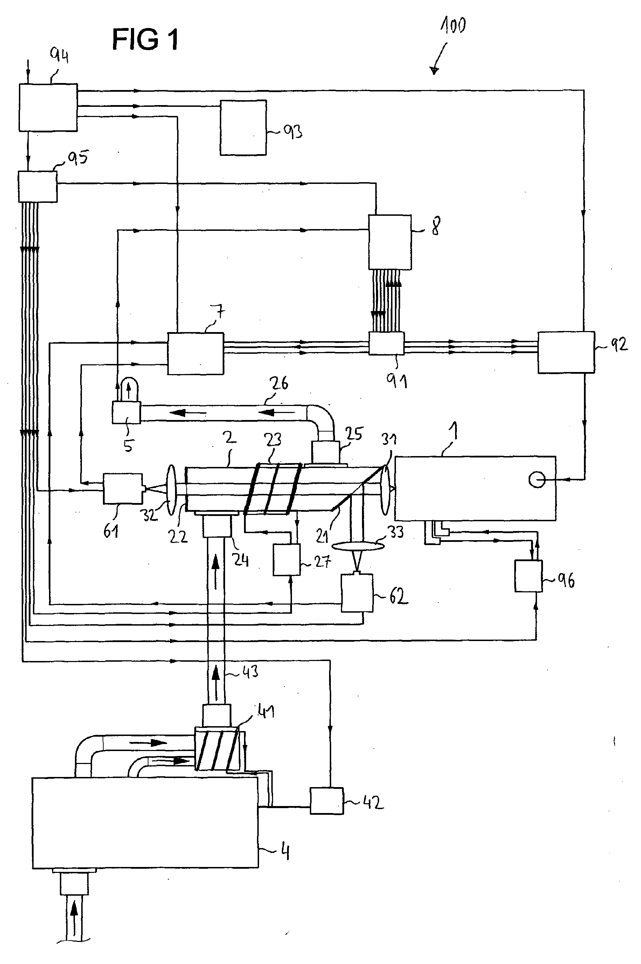

Die Figur 1 zeigt eine Messvorrichtung 100 zur Untersuchung eines Probegases mittels Infrarot-Absorptionsspektroskopie. Die Vorrichtung 100 umfasst einen Laser 1, eine Messkammer 2, zwei Detektoren 61, 62, eine Vorkammer 4, ein Spirometer 5, einen Ladungsverstärker 7 und eine Auswerteinheit 8.

Der Laser 1 ist ein Infrarot-Quantenkaskadenlaser (QCL), der eine Linienbreite unter 0,2 cm⁻¹ insbesondere eine Linienbreite von 0,05 cm⁻¹ aufweist. Die Grundfrequenz des Quantenkaskadenlasers wird über dessen Temperatur eingestellt. Diese wird auf einer Zeitskala von etwa 0,05 bis 0,5 s mittels einer Laser-Steuereinheit 92 geregelt. Die Laserfrequenz ist zusätzlich periodisch innerhalb eines definierten Spektralbereichs variierbar. Hierzu wird eine Spannung, die im Folgenden auch als "Sweep-Spannung" bezeichnet wird, mittels der Laser-Steuereinheit 92 zusätzlich am Quantenkaskadenlaser 1 angelegt. Die Sweep-Spannung und ein damit korrespondierender Sweep-Strom sorgen für eine kurzfristige Temperaturerhöhung während des zusätzlichen Stromflusses im Laser und verschiebt damit die Frequenz. Die Parameter des Lasers sind derart eingestellt, dass vorzugsweise direkt nach Beendigung des Stromflusses wieder die Grundfrequenz ausgegeben wird.

Die Sweep-Spannung wird kontinuierlich, beispielsweise mittels einer Dreiecksspannung oder Sägezahnspannung erhöht und dann wieder abgesenkt, wodurch eine kontinuierliche Frequenzvariation stattfindet. Aufsetzend auf der Grundfrequenz wird die Frequenz des Lasers 1 somit periodisch variiert. Die Frequenzvariation korreliert mit einer Durchstimmbarkeit des Lasers, die bei mindestens 0,5 cm⁻¹ liegt. Beispiele für die Breite der Durchstimmbarkeit sind 1, 2, 6, 20 oder 60 cm⁻¹. Die Durchstimmbarkeit gibt dabei einen Spektralbereich an, innerhalb dessen die Laserfrequenz variiert wird. Die Modulationsfrequenz, mit der die Laserfrequenz periodisch variiert wird, liegt im Bereich zwischen 1 und 500 Hz. Sie definiert, wie häufig der betrachtete Spektralbereich ausgemessen wird. Im Folgenden wird beispielhaft von einer Modulationsfrequenz von 50 Hz ausgegangen.

Der Laser 1 ist ein gepulster Laser, der Lichtsignale mit einer Pulsdauer von weniger als 200 ns, insbesondere von 100 ns oder sogar kürzer aussendet. Damit ist die maximale Zeitauflösung einer Messung auf 200 ns bzw. 100 ns begrenzt. Die Verwendung relativ kurzer Pulsdauern sorgt insofern für spektral schmale Linienbreiten, als bei lang anhaltenden Pulsen eine Linienverbreiterung auf Grund einer Temperaturerhöhung erfolgt, die mit einem vergleichsweise langen Aussenden von Laserlicht verbunden ist.

Die Laserrate, das heißt die Anzahl der Pulse, die pro Sekunde ausgesandt wird, liegt beispielsweise zwischen 10 und 100 kHz. Im Folgenden wird beispielhaft eine Laserrate von 50 kHz angenommen.

Der Laser 1 ist in einem geschlossenen Gehäuse angeordnet, das einen Kontakt mit der Außenluft verhindert. Hierzu ist er beispielsweise in einem TO3-Gehäus angeordnet. Eine Wasserkühlung 96 sorgt für eine Kühlung des Lasers 1.

Über die Laser-Steuereinheit 92 wird des Weiteren das Treibersignal für den Quantenkaskadenlaser 1 angelegt.

Die Messkammer 2 weist ein abgeschrägtes Eintrittsfenster 21, durch das das Laserlicht in die Messkammer 2 eintritt, und ein senkrecht zum Strahlengang angeordnetes Austrittsfenster 22 auf. Das vom Laser 1 ausgestrahlte Licht wird über eine antireflexbeschichtete Linse 31 auf das abgeschrägte Eintrittsfenster 21 gelenkt. Am Eintrittsfenster 21 wird das Licht in zwei Teilstrahlen aufgeteilt. Der transmittierte Strahl durchquert die Messkammer 2, verlässt die Messkammer 2 durch das Austrittsfenster 22 und fällt nach Fokussierung durch eine weitere antireflexbeschichtete Linse 32 auf einen ersten Detektor 61. Der reflektierte Strahl fällt über eine weitere antireflexbeschichtete Linse 33 auf einen zweiten Detektor 61. Die antireflexbeschichteten Linsen 31, 32, 33, die beispielsweise aus ZnSe, Saphir, CaF₂ oder BaF₂ bestehen, sind direkt mit dem Laser 1 bzw. den jeweiligen Detektoren 61, 62 verbunden, so dass der Messaufbau nur aus vier Komponenten besteht, nämlich dem Laser, den beiden Detektoren und der Messkammer. Dies führt zu einem einfachen, robusten Aufbau.

Das Laserlicht durchquert die Messkammer 1, die spiegellos ausgebildet ist, nur einmal. Dies erhöht weiter die Einfachheit und damit Störungsunanfälligkeit des Messaufbaus.

Die Messkammer 2 umfasst eine Temperiereinrichtung 23, die insbesondere als Heizeinrichtung ausgestaltet ist und die über einen Temperaturregler 27 geregelt wird.

Die Temperiereinrichtung 23 sorgt für eine konstante Temperatur innerhalb der Messkammer, die bei beispielsweise 35 °C oder darüber liegt. Dies verhindert, dass eventuell in dem Probegas, das die Messkammer 2 durchströmt, vorhandener Wasserdampf die Messkammer 2 beschlägt. Die konstante Temperatur kann auch unterhalb der Umgebungstemperatur liegen.

Zur Zuführung eines Probegases in die Messkammer 2 weist diese einen ersten Anschluss 24 auf. Der Anschluss 24 ist am Messkammergehäuse angeordnet, das beispielsweise aus Aluminium besteht. Über den Anschluss 24 wird der Messkammer 2 das Probegas von einer Vorkammer 4 über einen Schlauch 43 oder dergleichen zugeführt. Der Vorkammer 4 ist ebenfalls eine Heizeinrichtung 41 zugeordnet, die über einen Temperaturregler 42 eine Regelung der Temperatur des der Messkammer 2 zugeführten Probegases vornimmt. Hierdurch wird bereits in der Vorkammer 4 das Probegas erwärmt und in seinem Wasserdampfgehalt reduziert. Statt der Heizeinrichtung 41 könnte auch eine Temperiervorrichtung 41 verwendet werden, die das Probegas in der Vorkammer 4 bei Bedarf auch kühlen könnte.

Weiter weist die Messkammer 2 einen Anschluss 25 für das aus der Messkammer 2 ausströmende Probegas auf. Das Probegas strömt dabei beispielsweise durch einen Schlauch 26 oder dergleichen zu einem Spirometer 5, das den Volumenstrom, der durch die Messkammer 2 fließt, bestimmt. Nach Durchströmen des Spirometers 5 tritt das Probegas aus der Messvorrichtung in die Umgebung aus, wobei das Spirometer 5 auch an anderer Stelle in der Messvorrichtung angeordnet sein kann.

Das Probegas strömt in die Messkammer 2 senkrecht zu der Richtung, in der das Laserlicht die Messkammer 2 durchstrahlt. Ebenso strömt es senkrecht zur letztgenannten Richtung aus der Messkammer 2 wieder heraus. Dabei sind die Anschlüsse 24, 25 versetzt am Messkammergehäuse angeordnet.

Der gesamte Messaufbau der Messvorrichtung ist ein offener Aufbau ohne Ventile oder Luftklappen, die den Fluss des Probegases behindern könnten. Vielmehr kann das Probegas den beschriebenen Aufbau ungehindert durchströmen. Dabei ist vorgesehen, dass der Querschnitt in der Zuleitung 43, der Messkammer 2 sowie der Ableitung 26 im Wesentlichen konstant ist, so dass an allen Stellen ein laminarer Fluss gewährleistet ist und keine Gasansammlungen an bestimmten Stellen erfolgen. Vielmehr verdrängt Probegas, das über die Vorkammer 4 in die Messkammer 2 eintritt, komplett das zuvor vorhandene Probegas aus dem Messaufbau. Das Probegas strömt durch die Vorkammer 4 in die Messkammer 2 und von der Messkammer 2 durch das Spirometer 5 wieder aus der Messvorrichtung hinaus.

Die Messungen werden bei Normaldruck durchgeführt. Die Messkammer 2, die Vorkammer 4, die Zuleitung 43, die Ableitung 26 und das Spirometer 5 derart ausgeführt, dass sie bis zu einem Überdruck von bis zu 200 mbar im Vergleich zum Normaldruck dicht sind. Liegt kein Druckunterschied zwischen dem Gaseinlass 24 und dem Gasauslass 25 vor, kann das Probegas bis zu mehreren 10 Minuten unverändert in der Messkammer 2 verbleiben.

Die nachfolgend noch näher beschriebenen Infrarot-Absorptionsmessungen werden in jeder Phase des Gasflusses durch die Messkammer 2 durchgeführt, insbesondere auch, wenn das Probegas die Messkammer 2 durchströmt. Die durchgeführten Messungen erfolgen in echtem Durchfluss. Aufgrund des offenen Aufbaus der Messkammer 2 kann das Probegas in der Messkammer 2 dabei beliebig schnell ausgetauscht werden.

Wie noch ausgeführt wird, ist die beschriebene Messvorrichtung dazu geeignet und einrichtbar, als Probegas das Atemgas eines Menschen oder Tieres zu messen. Im Falle der Verwendung von Atemgas als Probegas sorgt der Atemdruck dafür, dass der neue Atemzug den alten Atemzug aus der Messkammer verdrängt und dabei der neue Atemzug in Echtzeit gemessen wird. Somit wird die Atemgasprobe allein durch die Atmung für jeden Patienten individuell so schnell wie nötig in der Messkammer ausgetauscht, wobei Messungen in Echtzeit im Durchfluss erfolgen. Der Atemwiderstand des Messgerätes ist dabei derart ausgelegt, dass er für den Gasfluss bei weniger als 60 mbar liegt.

Bei den Detektoren 61, 62 handelt es sich um MCT-Detektoren. Solche Detektoren sind Halbleiterdetektoren auf der Basis von Quecksilber(II)Cadmium(II)Tellurit. Die Detektoren 61, 62 sind bevorzugt peltiergekühlt, wodurch ein Verzicht auf mit flüssigem Stickstoff gekühlte Detektoren möglich ist, bei trotzdem hoher Sensitivität. Der Verzicht auf flüssigem Stickstoff zur Kühlung erweitert den Einsatzbereich der Messvorrichtung, beispielsweise im klinischen Alltag.

Beide Detektoren 61, 62 werden quasi zeitgleich ausgelesen. Jeder Detektor 61, 62 misst dabei das gesamte Spektrum des durch den Laser 1 ausgesandten Lichts. Hierdurch werden Fehler durch Variation der Detektorsensibilität von Detektor zu Detektor vermieden.

Das von den Detektoren 61, 62 ausgelesene Signal wird zunächst in einem Leitungsverstärker 7 für jeden Detektor 61, 62 getrennt verstärkt und integriert. Das verstärkte Signal wird dann jeweils über einen Adapter 91 einer Auswerteinheit 8 zugeführt, die beispielsweise mittels eines üblichen PCs und geeigneter Software realisiert ist. Das Signal des Detektors 62 dient dabei allein der Normierung der Signalstärke. So können Intensitätsschwankungen am Detektor 61, die durch Intensitätsschwankungen des Lasers 1 verursacht sind, mittels des Signals des Detektors 62 korrigiert werden. Dies erhöht die Genauigkeit der Auswertung.

Die Auswerteinheit 8 empfängt des Weiteren Signale vom Spirometer 5. Auch kann vorgesehen sein, dass der Auswerteinheit 8 über einen nicht dargestellten Sensor oder die Temperaturregler 42, 27 die Temperatur des Probegases und/oder die Temperatur in der Messkammer 2 mitgeteilt wird. Der Auswerteinheit 8 ist ein Monitor 95 zugeordnet.

Die Auswerteinheit 8 erzeugt Steuersignale an die Laser-Steuereinheit 92 in Bezug auf das Sweep-Signal, die Lasertemperatur und die Triggerfrequenz. Der Monitor 95 kann dazu dienen, eine Auswertung der vorgenommenen Absorptionsmessungen graphisch darzustellen. Die Auswerteinheit 8 kann des Weiteren an ein Telekommunikationsnetz, z.B. das Internet und/oder ein Telefonnetz angeschlossen sein.

Ein Netzteil 95, das über einen Transformator 94 an einen Netzstecker angeschlossen ist, sorgt für eine elektrische Versorgung der verschiedenen Elemente der Messvorrichtung.

Wie ausgeführt, wird die Frequenz des Lasers 1 periodisch variiert. Der dabei durchfahrene Spektralbereich wird durch die Sweep-Spannung festgelegt. Bei einer Laserrate von 50 kHz und einer Sweep-Spannung von 50 Hz können pro durchfahrenen Spektralbereich 1.000 Laserpulse gemessen werden. Der betrachtete Spektralbereich wird dabei 50 mal pro Sekunde ausgemessen. Die Messungen können über eine bestimmte Zeit, beispielsweise über die Länge eines Atemzuges gemittelt werden.

Der Detektor 61 ist derart ausgebildet, dass er eine einzelne Absorptionsmessung innerhalb von 10⁻⁵ Sekunden oder schneller durchführt, insbesondere innerhalb von 10⁻⁶ Sekunden oder schneller. Ein gesamter Frequenzbereich, d. h. der Frequenzbereich des durch die Sweep-Spannung definierten Spektralbereichs, kann in etwa 0,002 bis 1 Sekunde abgetastet werden. Im genannten Ausführungsbeispiel liegt die Punktdichte pro Spektralbereich (pro Sweep) bei 1.000 Punkten. Jeder Laserpuls wird detektiert und in einen Messpunkt umgewandelt. Alternativ kann die Punktdichte auch geringer gewählt werden, bei etwa 500 Punkten oder auch nur 20 Punkten pro Spektralbereich.

Die hohe Punktdichte beim Durchfahren des durchmessenden Spektralbereichs verbunden mit der kleinen spektralen Linienbreite des Lasers 1 ermöglicht eine spektrale Auflösung von kleiner als 0,02 cm⁻¹. Dies bedeutet, dass die Absorptionsbanden der vermessenen Gasprobe sehr genau aufgenommen und analysiert werden können. Durch Entfaltung oder andere mathematische verfahren kann die spektrale Auflösung ggf. noch weiter verbessert werden.

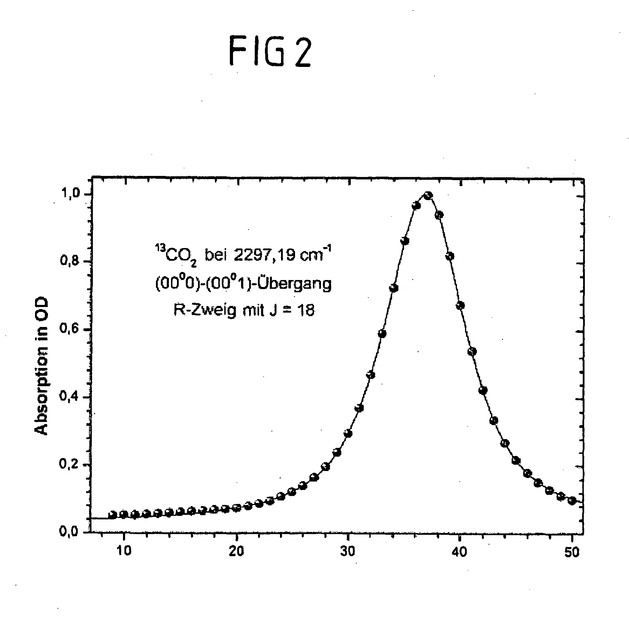

Die Figur 2 zeigt die Messung einer ¹³CO₂-Absorptionslinie, die bei einer Frequenz (in Wellenzahlen) von 2.297,19 cm⁻¹ liegt. Die Abzisse gibt die Änderung der Frequenz (aufgrund der Sweep-Spannung) gegenüber der Grundfrequenz des Lasers 1 an. Die Absorption ist in OD (optische Dichte) angegeben. Die Punktdichte ist hoch genug, um die Absorptionslinie sehr genau bestimmen zu können. Es kann vorgesehen sein, die Kurve zu fitten. Eine Anpassung der Absorptionslinie kann beispielsweise mit Lorenzkurven erfolgen.

Die Datenaufnahme erfolgt jeweils mittels einer Analog/Digital-Karte, die in der Auswerteinheit 8 angeordnet ist und pro Mikrosekunde einen Datenpunkt oder mehr aufnimmt. Die Auflösung ist dabei besser als 12 bit.

Bei einer Laserrate von 50 kHz, einer Modulationsfrequenz des Sweep-Stroms von 50 Hz werden 50 Spektren pro Sekunde gemessen. Pro Spektrum werden 1.000 Punkte gemessen.

Die Ausleserate der Detektoren 61, 62 ist bevorzugt derart gewählt, dass sie doppelt so groß ist wie die Laserrate. Bei einer Laserrate von 50 kHz beträgt die Ausleserate der Detektoren 61, 62 somit 100 kHz. Dies führt dazu, dass nur bei jedem zweiten Auslösevorgang ein detektiertes Lichtsignal ausgelesen wird. Die dazwischen stattfindenden Messungen betreffen den Hintergrund bzw. Rauschen. Das Auslesen der Detektoren 61, 62 mit der doppelten Laserrate ermöglicht es, den Hintergrund bei jeder Lichtmessung sofort abzuziehen. Dies erfolgt bevorzugt in der Auswerteinheit 8 und erhöht weiter die Genauigkeit der Messung.

Es werden mit der beschriebenen Messvorrichtung Absorptionsspektren für einen definierten Spektralbereich in Echtzeit gemessen, da pro Sekunde eine Mehrzahl von Spektren aufgenommen werden kann. Wahlweise kann dabei über eine Mehrzahl von Spektren gemittelt werden, was die Genauigkeit der Messung weiter erhöht. Es wird damit in Echtzeit eine Detektion von Veränderungen der Zusammensetzung eines Probegases möglich.

Um eine hohe Genauigkeit zu erhalten, ist eine hohe Frequenzstabilität erforderlich. Dies wird dadurch erreicht, dass die Temperatursteuerung der Laser-Steuereinheit 92 von einer Messsoftware nachgesteuert wird. Dabei wird die Temperatur in kleinen Schritten nachgeregelt, so dass immer dasselbe Gasabsorptionsmaximum (zum Beispiel von ¹²CO₂) an der gleichen Position im Messbereich liegt. Des Weiteren ist vorgesehen, dass auch die Sweep-Spannung nachgeregelt wird, so dass die weiteren Absorptionslinien an der gewünschten Position im Messbereich liegen. Dadurch werden die Messungen optimal reproduzierbar und können gemittelt werden. Durch große Mittelungszahlen wird das Signal-Rausch-Verhältnis verbessert. Auch erlaubt die Messsoftware bevorzugt eine automatische Erkennung, ob die Laserleistung nachlässt und wann der Laser 1 ausfällt. Die entsprechende Messsoftware kann Teil der Auswerteinheit 8 oder der Laser-Steuereinheit 92 sein.

Weiter kann vorgesehen sein, dass eine schnelle Auswertung der Spektren über eine Integration von speziellen Frequenzbereichen erfolgt, die einzelnen Absorptionslinien zuzuordnen sind. Die Aufnahme von Eichgraden mit Gasproben, die in ihrer Zusammensetzung bekannt sind, erlaubt dabei die einfache und genaue Bestimmung des Offsets ohne ein Fitten der Daten. Da die Frequenzstabilität reproduzierbar eingestellt ist, können gezielt Frequenzbereiche adressiert werden, aus denen die Konzentrationen hochgenau bestimmt werden. Ein Fitten der großen Datenmengen würde möglicherweise den Messprozess verlangsamen und ist daher nicht zwingend erforderlich.

Durch die hohe Selektivität der Messung kann die Messung unabhängig von Testgasen wie Sauerstoff oder anderen Narkosegasen erfolgen. Mit der vorhandenen hohen spektralen Auflösung können Einflüsse jedes anderen Gases abgetrennt werden.

Die Messvorrichtung ist im dargestellten Ausführungsbeispiel dazu optimiert, Leberleistungsmessungen nach Verabreichung von ¹³C-markiertem Methacetin durchzuführen. Das zusätzlich metabolisierte ¹³CO₂ wird in der Atemluft nachgewiesen. Dies ist ausführlich in der WO 2007/000145 A2 beschrieben, auf die insofern Bezug genommen wird.

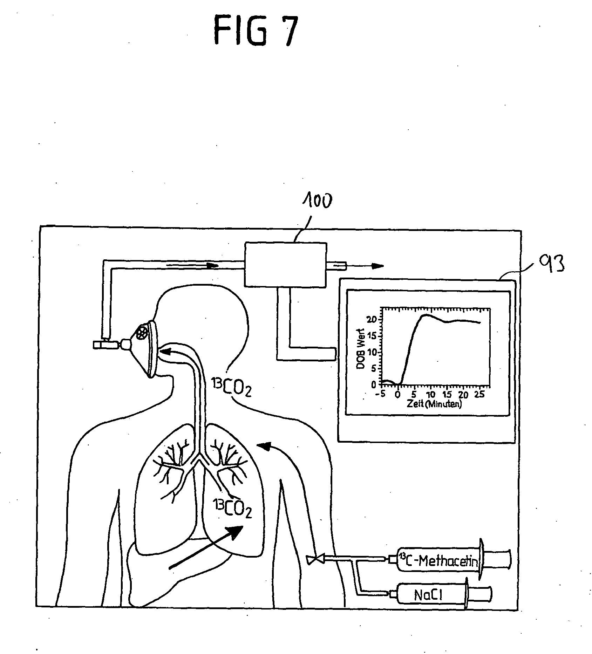

Wie in der Figur 7 schematisch dargestellt ist, trägt ein zu untersuchender Patient eine Atemmaske, die ein Lufteinlassventil und ein Luftauslassventil aufweist. Dadurch wird die Einatemluft von der Ausatemluft getrennt. Ausgeatmete Luft kann nicht wieder eingeatmet werden. Am Luftauslassventil wird ein Plastikschlauch angeschlossen, der die gesamte Atemluft an die Messvorrichtung 100 leitet und der mit der Vorkammer 4 verbunden ist. Die Maske und die Schläuche sind ebenfalls bis zu einem Überdruck von bis zu 200 mbar dicht, so dass die gesamte Atemluft durch die Messvorrichtung 100 strömt.

Dabei erkennt eine Messsoftware automatisch, falls die Maske nicht richtig auf dem Patientengesicht sitzt bzw. verrutscht ist. Auch erkennt die Messsoftware, ob der Patient noch atmet oder nicht und gibt gegebenenfalls eine Warnung aus. Des Weiteren ist vorgesehen, dass die Messsoftware erkennt, wann die Messung beendet werden kann. Eine solche Messsoftware kann ebenfalls in die Auswerteinheit 8 integriert sein.

Die Laserfrequenz des Lasers 1 und der durch die Sweep-Spannung definierte Spektralbereich sind nun so gewählt, dass mindestens zwei Absorptionslinien des Probegases in dem definierten Spektralbereich liegen. Im betrachteten Beispiel sind dies eine Absorptionslinie von ¹³CO₂ und eine Absorptionslinie von ¹²CO₂. Nach Verabreichung von ¹³C-markiertem Methacetin wird dieses in der Leber abgebaut und ist in der Atemluft nachweisbar. Der Abbau korreliert mit einem Anstieg von ¹³CO₂ in der Atemluft, was zu einem veränderten Verhältnis von ¹³CO₂ zu ¹²CO₂ führt. Das Verhältnis wird durch die Messvorrichtung 100 anhand der Absorptionmessungen bestimmt und dessen zeitlicher Verlauf auf einem Monitor 93 dargestellt.

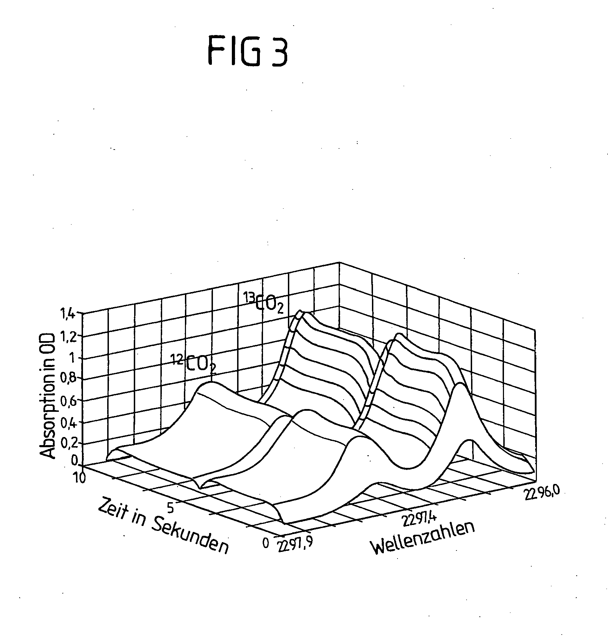

Die Figur 3 zeigt die gleichzeitige Messung der ¹²CO₂ und ¹³CO₂ Absorptionslinien im Verlauf von zwei aufeinander folgenden Atemzügen. Die Absorptionsänderung ist in OD (Optische Dichte) angezeigt. Sie ist sowohl gegen die Zeit in Sekunden als auch gegen die Frequenz in Wellenzahlen aufgenommen und dargestellt. Man sieht deutlich die starken Variationen der Absorption und damit der Konzentration.

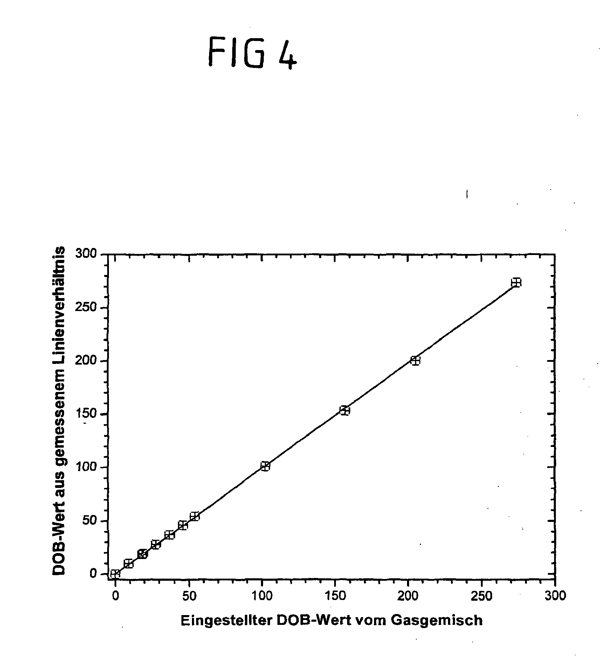

Die Figur 4 verdeutlicht die Genauigkeit, mit der das Verhältnis der ¹³CO₂/¹²CO₂ Konzentration gemessen werden kann. Die Konzentrationsverhältnisse wurden mit sehr genau charakterisierten Testgasen eingestellt und diese Verhältnisse mittels Testmessungen verifiziert. Die Abszisse zeigt dabei einen durch Testgase eingestellten DOB-Wert. Die Ordinate zeigt den gemessenen DOB-Wert, der aus den Linienverhältnissen gemäß Figur 3 bestimmt wird. Dabei bezeichnet 1 DOB eine Änderung des ¹³CO₂ zum ¹²CO₂ Verhältnisses um ein Tausendstel über dem natürlichen Verhältnis. Es zeigt sich, dass das Verhältnis der ¹³CO₂ zur ¹²CO₂ Konzentration mit einer Genauigkeit von besser als 5 DOB pro Atemzug - bei Messung von mehreren Atemzügen in Folge - bestimmt werden kann.

Der Messbereich erstreckt sich von 0 DOB bis über 1000 DOB im Konzentrationsbereich von 0,08% bis 8% CO₂. Über den gesamten Bereich wird das Verhältnis mit höchster Genauigkeit gemessen.

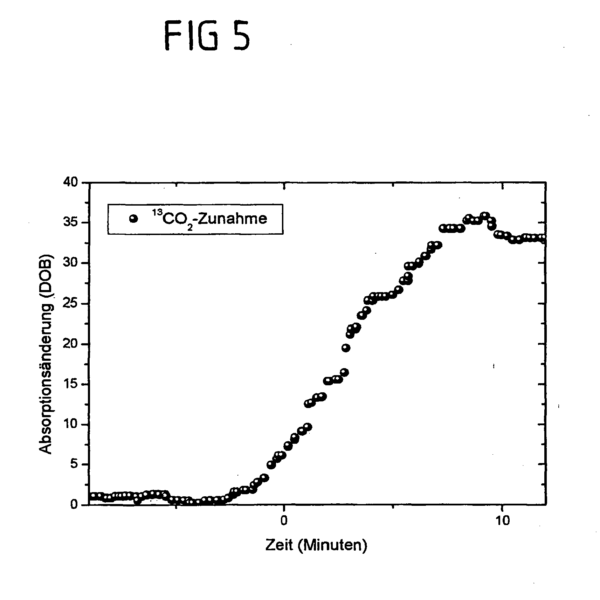

Die Figur 5 zeigt die Leberleistungsbestimmung nach Einnahme von ¹³C-markiertem Methacetin. Dargestellt ist die ¹³CO₂-Zunahme eines Probanden nach Einnahme, die mit einer erhöhten Absorptionsänderung der ¹³CO₂ Absorptionslinie einhergeht. Jeder Atemzug wurde vermessen und entspricht einem Messpunkt (d.h. die bei einem Atemzug durchgemessenen Spektren und das aus diesen ermittelte Verhältnis wurden zu einem Messpunkt gemittelt). Der Anstieg und das Maximum der Absorptionsänderung sind klar und quantitativ genau bestimmbar. Das Diagnostikum wurde etwa bei -3 Minuten verabreicht.

In entsprechender Weise kann auch das Verhältnis anderer Isotope, Elemente oder Moleküle bestimmt werden.

Die Messvorrichtung erlaubt neben der Messung des Verhältnisses bestimmter Isotope auch andere Auswertungen. Beispielsweise kann die Gesamtmenge eines Stoffwechselprodukts, beispielsweise von ¹³CO₂ im Atemzug gemessen werden. So wird der Volumenstrom, der durch die Messkammer 2 fließt, mit dem Spirometer 5 bestimmt. Da das Volumen der Messkammer 2 konstant ist und die Absorption zeitaufgelöst bestimmt wird, kann die Kohlendioxidmenge, die durch die Messkammer fließt, durch Integration über der Zeit bestimmt werden. Insbesondere kann aus der Absorption direkt die Konzentration bestimmt werden kann, da der Extinktionskoeffizient für jede Absorptionslinie bekannt ist, ebenso wie die Länge der Messkammer. Da die Messvorrichtung es ermöglicht, die Absorption zeitaufgelöst in Echtzeit und ebenfalls den Volumenstrom zeitaufgelöst in Echtzeit zu verfolgen, kann die Gesamtmenge über eine Integration des Produktes von Volumen und Konzentration über der Zeit in Echtzeit bestimmt werden.

Durch die Messung der ¹³CO₂ Konzentration und der ¹²CO₂ Konzentration kann somit die Kohlendioxidmenge, die in der Messkammer ist, getrennt für ¹³CO₂ und ¹²CO₂ bestimmt werden. Insbesondere kann die Gesamtmenge von ¹³CO₂ - bei Messung von mehreren Atemzügen in Folge - auf etwa 10 µg genau pro Atemzug bestimmt werden.

Eine weitere Anwendung bestimmt die Erfassung der Veränderung der CO₂ Absorptionslinien bei variierender Konzentration des CO₂ in der Atemluft, bei gleichbleibendem Druck in der Atemluft. Mit zunehmender Gaskonzentration und/oder sich veränderndem Partialdruck ändert sich die Linienbreite des Absorptionslinien mittels an sich bekannter Linienverbreiterungsmechanismen. Die Linienbreite kann bei unterschiedlichen bekannten CO₂ Konzentrationen mittels der Messvorrichtung gemessen werden, vergleiche die Absorptionslinien der Figuren 2 und 3.

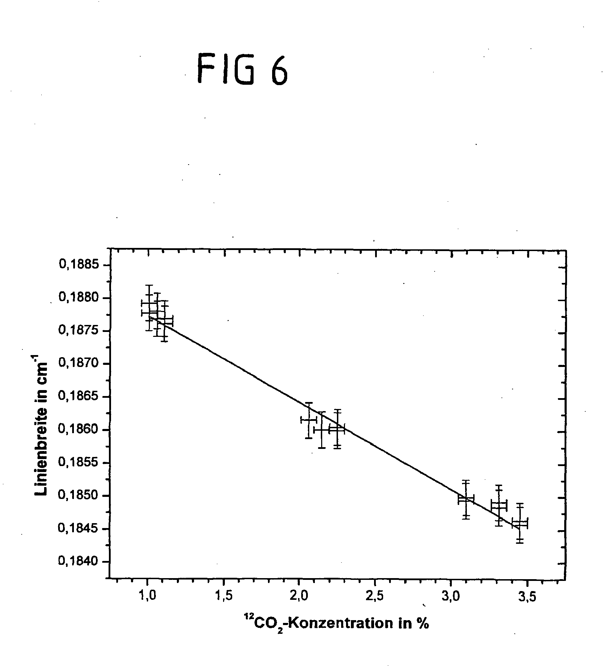

Die Figur 6 zeigt die gemessene Linienbreite in Abhängigkeit von der ¹²CO₂ Konzentration des Atemgases in Prozent. Die ermittelte Abhängigkeit kann zur weiteren Fehlerreduktion ausgewertet werden.

Der Frequenzbereich für die Messung von ¹³CO₂ und ¹²CO₂ liegt zwischen 2200 und 2400, insbesondere bei 2295 bis 2305 cm⁻¹ Allgemein wird bevorzugt ein Laser 1 verwendet, der im Frequenzbereich zwischen 2 µm bis 12 µm Licht emittiert.

Der Einsatz der beschriebenen Messvorrichtung ist nicht auf die Messung des CO₂ Gehaltes der Atemluft beschränkt. Mit dem beschriebenen Messgerät kann jede beliebige Gasprobe untersucht werden. Dabei kann z.B. ein Isotopenverhältnis beliebiger Gase hochempfindlich und sehr genau in Echtzeit bestimmt werden. Die erfindungsgemäße Messvorrichtung ermöglicht eine quantitative, dynamische und zeitaufgelöste Messung von Stoffwechselparametern in Echtzeit. Dabei können auch Belastungsanalysen eines Menschen oder Tieres in Echtzeit durchgeführt werden.

## Patentansprüche

1. Messvorrichtung (100) zur Untersuchung eines Probegases mittels Infrarot-Absorptionsspektroskopie, mit einer Messkammer (2) und einer Vorkammer (4), die zur Homogenisierung eines in der Messvorrichtung (100) zumessenden Probegases dient und mindestens zwei Abzweigungen unterschiedlicher Länge und unterschiedlichen Durchmessers aufweist,
wobei die Abzweigungen im bestimmungsgemäßen Betrieb der Vorkammer (4) von Teilen des Probegases durchlaufen werden, wobei die Teile des Probegases nach den Abzweigungen wieder zusammengeführt werden, so dass das zusammengeführte Probegas über einen Schlauch (43) oder dergleichen der Messkammer (2) wird zugeführt wird,
wobei die Durchmesser der einzelnen Abzweigungen derart ausgewählt sind, dass eine zweite Abzweigung einen um mindestens 50 % größeren Durchmesser aufweist als eine erste Abzweigung und
wobei die Summe der Querschnitte der einzelnen Abzweigungen einen größeren oder gleich großen Strömungsquerschnitt wie der Rest der Messvorrichtung (100) aufweist.

2. Messvorrichtung (100) nach Anspruch 1, **dadurch gekennzeichnet, dass** mehrere Abzweigungen den gleichen Durchmesser aufweisen.

3. Messvorrichtung (100) nach einem der vorangehenden Ansprüche, **dadurch gekennzeichnet, dass** die Vorkammer (4) dazu ausgebildet ist, das Probegas auf eine bestimmte Temperatur zu erwärmen oder abzukühlen und dadurch den Wasserdampfgehalt des Probegases zu reduzieren.

4. Messvorrichtung (100) nach einem der vorangehenden Ansprüche, **dadurch gekennzeichnet, dass** die Vorkammer (4) dazu ausgebildet ist, den Wasserdampfgehalt des Probegases auf wenigstens 60% relative Luftfeuchtigkeit zu reduzieren.

5. Messvorrichtung (100) nach einem der vorangehenden Ansprüche, **dadurch gekennzeichnet, dass** die Vorkammer (4) eine semipermeable Membran aufweist, die ausschließlich einen Austausch von Wasserdampf, nicht jedoch von anderen Substanzen, erlaubt.

6. Messvorrichtung (100) nach Anspruch 5, **dadurch gekennzeichnet, dass** eine aktive Fläche der semipermeablen Membran mehr als 150 cm² beträgt.

## Claims

1. Measurement device (100) for analyzing a sample gas by infrared absorption spectroscopy, comprising a measurement chamber (2) and an ante-chamber (4) which serves for homogenization of a sample gas to be measured in the measurement device (100) and which comprises at least two branchings of different length and different diameter,
wherein parts of the sample gas pass through the branchings in normal mode of the ante-chamber (4), wherein the parts of the sample gas will be brought together after the branchings such that the joined sample gas is supplied to the measurement chamber (2) via a tube (43) or the like,
wherein the diameter of the several branchings are selected such that a second branching has a diameter which is at least 50% larger than a first branching, and
wherein the sum of the cross sections of the branchings has a flow cross section which is larger than or equal to the cross section of the rest of the measurement device (100).

2. Measurement device (100) according to claim 1, **characterized in that** multiple branchings have the same diameter.

3. Measurement device (100) according to any of the preceding claims, **characterized in that** the ante-chamber (4) is designed to heat or cool the sample gas onto a defined temperature and to reduce in this way the water vapor content of the sample gas.

4. Measurement device (100) according to any of the preceding claims, **characterized in that** the ante-chamber (4) is designed to reduce the water vapor content of the sample gas to at least 60% relative air humidity.

5. Measurement device (100) according to any of the preceding claims, **characterized in that** the ante-chamber (4) comprises a semipermeable membrane which exclusively allows an exchange of water vapor but does not allow an exchange of other substances.

6. Measurement (100) device according to claim 5, **characterized in that** an active area of the semipermeable membrane is more than 150 cm².

## Revendications

1. Dispositif de mesure (100) servant à étudier un échantillon gazeux au moyen d'une spectroscopie d'absorption dans l'infrarouge, comprenant une chambre de mesure (2) et une préchambre (4), qui sert à l'homogénéisation d'un échantillon gazeux à mesurer dans le dispositif de mesure (100) et qui présente au moins deux embranchements de longueur et de diamètre différents,
dans lequel les embranchements sont traversés, lorsque la préchambre (4) fonctionne conformément à son usage, par des parties de l'échantillon gazeux, dans lequel les parties de l'échantillon gazeux sont à nouveau réunies en aval des embranchements de sorte que l'échantillon gazeux réuni est acheminé, par l'intermédiaire d'un tuyau flexible (43) ou d'un dispositif similaire, à la chambre de mesure (2),
dans lequel les diamètres des divers embranchements sont choisis de telle manière qu'un deuxième embranchement présente un diamètre plus grand d'au moins 50 % que le premier embranchement, et
dans lequel la somme des sections transversales des divers embranchements présente une section transversale d'écoulement plus grande ou égale comparée au reste du dispositif de mesure (100).

2. Dispositif de mesure (100) selon la revendication 1, **caractérisé en ce que** plusieurs embranchements présentent le même diamètre.

3. Dispositif de mesure (100) selon l'une quelconque des revendications précédentes, **caractérisé en ce que** la préchambre (4) est réalisée pour réchauffer ou refroidir l'échantillon gazeux à une température définie et ce faisant pour réduire la teneur en vapeur d'eau de l'échantillon gazeux.

4. Dispositif de mesure (100) selon l'une quelconque des revendications précédentes, **caractérisé en ce que** la préchambre (4) est réalisée pour réduire la teneur en vapeur d'eau de l'échantillon gazeux à une teneur égale à au moins 60 % d'humidité de l'air relative.

5. Dispositif de mesure (100) selon l'une quelconque des revendications précédentes, **caractérisé en ce que** la préchambre (4) présente une membrane semi-perméable, qui permet exclusivement un échange de vapeur d'eau, et donc ne permet pas en revanche l'échange d'autres substances.

6. Dispositif de mesure (100) selon la revendication 5, **caractérisé en ce qu'**une surface active de la membrane semi-perméable est supérieure à 150 cm².
